# EUROPEAN PATENT SPECIFICATION

(11) **EP 2 000 034 B1**
(45) Date of publication and mention of the grant of the patent: **02.01.2019**
(21) Application number: 07736962.7
(22) Date of filing: 27.03.2007
(51) Int. Cl.: A23F 5/18, C07C 67/52, C07C 69/732, A61K 36/74, A23L 33/10

(54) **METHOD FOR PRODUCTION OF CHLOROGENIC ACID-CONTAINING MATERIAL**
VERFAHREN ZUR HERSTELLUNG EINER CHLOROGENSÄUREHALTIGEN SUBSTANZ
PROCEDE DE PRODUCTION D'UNE SUBSTANCE CONTENANT DE L'ACIDE CHLOROGENIQUE

(30) Priority: 28.03.2006 JP 2006087127
(43) Date of publication of application: 10.12.2008
(73) Proprietor: Kao Corporation, Chuo-ku Tokyo 103-8210 (JP)
(72) Inventor: KOYAMA, Shingo, Kamisu-shi, Ibaraki 314-0103 (JP); SATO, Hitoshi, Kamisu-shi, Ibaraki 314-0103 (JP)
(74) Representative: Hoffmann Eitle
(86) International application number: PCT/JP2007/000305
(87) International publication number: WO 2007/122796

(56) References cited:
- EP-A1- 0 340 354
- EP-A1- 1 559 421
- WO-A1-2006/093114
- JP-A- 2 265 433
- JP-A- 04 145 048
- JP-A- 04 145 049
- JP-A- 61 187 748
- JP-A- 2004 081 053
- JP-A- 2004 194 515
- JP-A- 2005 263 632
- JP-A- 2006 174 746
- JP-A- 2006 174 746
- US-A- 4 547 378

## Description

### Field of the Invention

The present invention relates to a method for production of a chlorogenic acid-containing material having a less disagreeable taste and less unpleasant odor.

### Background of the Invention

These days, people's interest in physiological effects of various ingredients contained in foods is growing much more than ever before. In Japan, the Ministry of Health, Labour and Welfare is supposed to grant permission to a food product (labeled as "food for specified health use") containing ingredients associated with such physiological functions or biological activities. These food products are commercialized in many processed forms, such as beverages, yogurt, soup, miso soup and hamburgers, as well as in other forms, such as chewable tablets and tablets . In Japan, people are recommended to ingest at least one of these food products 1-2 times per day.

Various materials are already suggested as having physiologically active functions. One such material is polyphenol, which is said to have physiologically active functions, such as antioxidative action, antihypertensive action and hepatic function ameliorating action (Patent Documents 1 and 2). In particular, the antihypertensive action of polyphenol is attracting more attention, and some products containing polyphenol having such action are permitted to carry the label "food for specified health use". Among them, there is a technology designed to incorporate chlorogenic acids into low salt soy sauce having potent antihypertensive action (Patent Document 3).

Coffee beans are mentioned as containing a large amount of chlorogenic acids. Nonetheless, coffee beans have limitations when used as ingredients for many food products and cosmetic products, because coffee beans and coffee bean extracts contain caffeine, and because these are not free from a disagreeable taste and unpleasant odor such as an acrid taste and grassy odor originating from green coffee beans, or from the burnt odor/bitter taste originating from roasted coffee beans . The illustrative techniques that have been known for removing caffeine from coffee beans or coffee bean extracts include a method of using activated carbon (Patent Document 4) . Meanwhile, the illustrative techniques that have been known for removing disagreeable taste and unpleasant odor include a method of contacting the coffee beans or coffee bean extract with microorganisms and optionally organic solvents (Patent Document 5). Furthermore, a number of techniques related to extraction treatment involving various solvents have been suggested (Patent Documents 6 to 8).
[Patent Document 1] JP-A-09-084565
[Patent Document 2] JP-A-2003-128560
[Patent Document 3] JP-A-2004-194515
[Patent Document 4] JP-A-57-012952
[Patent Document 5] JP-A-2004-081053
[Patent Document 6] JP-A-53-145963
[Patent Document 7] JP-A-02-265433
[Patent Document 8] JP-A-60-259145

US 4547378 concerns a roasted coffee extract decaffeination method providing a soluble coffee of improved flavour. In this method, roasted coffee extract is contacted with a caffeine solvent so that caffeine and a lesser amount of non-caffeine solubles is transferred to the solvent. The two liquids are separated and the caffeine solvent is typically concentrated. The concentrated solvent is then contacted with an aqueous caffeic acid suspension so that caffeic acid/caffeine complex crystals grow in the water phase. The complex crystals are filtered and the then at least partially decaffeinated caffeine solvent is separated from the water phase. Contact of the caffeine solvent and an aqueous caffeic acid suspension may be repeated to achieve further decaffeination. Then, the solvent, which contains substantially only non-caffeine solubles, is added to the substantially decaffeinated roasted coffee extract. The caffeine solvent is then stripped from the roasted coffee extract, which is subsequently dried.

EP 0340354 relates to a method of decaffeination wherein a caffeine-containing coffee extract solution is contacted with caffeic acid in the presence of water. The caffeine and the caffeic acid form an insoluble caffeine/caffeic acid complex wherein the complex is separated from the coffee extract solution.

### Summary of the Invention

The present invention provides a method for production of a chlorogenic acid-containing material, comprising contacting a coffee bean extract (A), which has been extracted from coffee beans using water or a mixture of a water miscible organic solvent and water at a mass ratio of water-miscible organic solvent/water of less than 70/30, with a mixture of a water-miscible organic solvent (B) and water (C), under such conditions that the mass ratio of the components (B) and (C) obtained after the mixture is mixed with the component (A) becomes 70/30 to 99.5/0.5; recovering a solid portion; and adding water (C) or a mixture having a mass ratio of water-miscible solvent (B)/water (C) of 5/95 or greater and less than 70/30 to the recovered solid portion, to dissolve the solid portion or to extract chlorogenic acids.

The present invention also provides a method for preparing a food product using the chlorogenic acid-containing material obtained as described above, wherein the food product contains 0.005 to 10% by mass of chlorogenic acids.

### Detailed Description of the Invention

Among the conventional techniques designed to remove disagreeable taste from a coffee bean extract, the method of contacting the coffee bean extract with microorganisms is facing problems such as sterilization after the treatment and the burden of the processes for bacterial body separation or the like. Meanwhile, the extraction treatment involving various solvents has a problem that the disagreeable taste and the unpleasant odor are both difficult to reduce sufficiently.

Therefore, the present invention is to provide a method for production of a chlorogenic acid-containing material which contains a coffee bean extract as a raw material, has reduced disagreeable taste and unpleasant odor stemming from coffee beans, and can be incorporated into a variety of food products.

The present inventors conducted investigation on a method for treating a coffee bean extract, and as a result, found that ingredients having disagreeable taste and unpleasant odor can be removed by extraction, while maintaining large amounts of chlorogenic acids in the solid portion, by treating the coffee bean extract with a mixture of a water-miscible solvent and water.

According to the method for production of the present invention, a chlorogenic acid-containing material having less disagreeable taste and less unpleasant odor can be obtained by a simple and convenient method.

As the coffee beans which may be used as the raw material of the coffee bean extract (A) used in the present invention, there may be mentioned Arabica coffee beans such as Mocha, Colombia and Brazil, Robusta coffee beans such as Java Robusta, AP-1 and Vietnam Robusta, or coffee beans obtained from hybrids thereof.

As the coffee bean extract (A) used in the present invention, products extracted from coffee beans using water or a mixture of water and a water-miscible organic solvent with a mass ratio of the water-miscible organic solvent/water of less than 70/30, by methods such as column extraction, stirring extraction and the like, are be used. During the extraction, an organic acid or an organic acid salt such as sodium ascorbate may also be added in advance to water or the mixture of water and a water-miscible organic solvent. As for the method for extraction, a method of introducing coffee beans into water, a mixture of water and a water-miscible organic solvent or the like, heating and stirring the mixture, and recovering an extract (batch method); a method of extracting by passing water or a mixture of water and a water-miscible organic solvent, through a column packed with coffee beans under high temperature and high pressure conditions (column method); and the like may be mentioned. A method of extracting under a so-called non-oxidative atmosphere, while removing dissolved oxygen through deaeration by boiling or aeration with an inert gas such as nitrogen gas, may also be used in combination. In order to increase the chlorogenic acidconcentration, the extract may be made into a paste form by performing evaporation and concentration or the like, or may be made into a powdered form by performing spray-drying, freeze-drying or the like.

As for the coffee bean extract, commercially available coffee bean extracts may also be used, instead of preparing an extract from coffee beans. Specifically, there may be mentioned "Flavor Holder CR-30R" from T. Hasegawa Co., Ltd. as the paste form, and "Raw Coffee Bean Extract P" from Oryza Oil & Fat Chemical Co., Ltd., "OXCH100" from Toyo Hakko Co., Ltd. and the like as the powdered form.

The coffee beans which are used as the raw material of the coffee bean extract (A) used in the method of the present invention, are preferably green coffee beans or lightly roasted coffee beans from the viewpoints of the chlorogenic acid content and reduction of a burnt odor/bitter taste. Specifically, it is preferable to use coffee beans having an L value (brightness) after roasting of 30 or greater, and more preferably 35 or greater, and it is even more preferable to use unroasted green coffee beans.

The L value (brightness) as used in the present invention is an index of the degree of roast determined from the color of the coffee beans, and can be measured using, for example, a colorimeter, such as Spectro Color Meter SE2000 (Nippon Denshoku Industries Co., Ltd.).

The coffee bean extract (A) used in the present invention contains one or more chlorogenic acids. The chlorogenic acids may exist as isomers and analogues, and thus include pure isomers, analogues or mixtures thereof. According to the present invention, chlorogenic acids refer to 3-caffeoylquinic acid, 4-caffeoylquinic acid, 5-caffeoylquinic acid (chlorogenic acid), 3, 4-dicaffeoylquinic acid, 3, 5-dicaffeoylquinic acid, 4,5-dicaffeoylquinic acid, 3-ferulylquinic acid, 4-ferulylquinic acid, 5-ferulylquinic acid, 3-ferulyl-4-caffeoylquinic acid and the like. In addition to these, caffeic acid, ferulic acid and the like may also be included. The chlorogenic acid-containing material refers to a material containing one or two or more species selected from these.

It is preferable that the chlorogenic acids are contained in the coffee beans in an amount of 2% by mass (hereinafter, simply expressed in "%") or greater, more preferably 4% or greater, and even more preferably 8% or greater, from the viewpoint that the burden of subsequent treatment processes is reduced. Furthermore, it is preferable that the chlorogenic acids are contained in the coffee bean extract in an amount of 5% or greater, more preferably 15% or greater, and even more preferably 30% or greater from the same viewpoint.

The water-miscible organic solvent (B) used in the present invention refers to an organic solvent which is soluble with water at any ratio, and examples thereof include ethanol, methanol, isopropyl alcohol, acetone, acetonitrile, and mixtures of two or more of these. Among them, alcohols such as ethanol, methanol and isopropyl alcohol, and mixtures of two or more of these are preferred from the viewpoint of the rate of extraction, and particularly upon considering the usage in food products, ethanol is preferred.

According to the present invention, from the viewpoints of improving the effect of eliminating disagreeable taste and unpleasant odor and improving the recovery rate of chlorogenic acids, it is necessary to adjust the mass ratio of the water-miscible organic solvent (B)/water (C) obtained after mixing the mixture of the water-miscible organic solvent (B) and water (C), in the range of 70/30 to 99.5/0.5, and it is preferable to adjust the mass ratio to 80/20 to 99/1, more preferably 90/10 to 98/2, even more preferably 90/10 to 97/3, even more preferably 91/9 to 96/4, and even more preferably 92/8 to 95/5. In this case, since there are also cases where component (B) and/or (C) is contained in component (A), it is preferable to measure the contents of the components (B) and (C) in the component (A) before contacting the mixture of the components (B) and (C) with the component (A), to determine the mass ratio of (B)/(C) to be mixed. Although the amounts of the component (B) and/or (C) contained in the component (A) are dependent on the above-describedproduction method or the form of the extract, since the component (B) is to be removed by a process of concentration or drying, the proportion of the component (B) in the extract is usually about 10% or less. The content of the component (C) in the component (A) is preferably 0 to 50%, more preferably 1 to 15%, even more preferably 2 to 8%, and even more preferably 3 to 7%.

As for the method of contacting the coffee bean extract (A) with a mixture of the water-miscible organic solvent (B) and water (C), it is preferable to carry out by introducing all the components in a vessel, mixing the components by shaking or stirring by means of a magnetic stirrer or a stirring blade, and the like.

According to the present invention, from the viewpoints of improving the recovery rate of chlorogenic acids and removing disagreeable taste and unpleasant odor, the content of chlorogenic acids in the total of the components (A), (B) and (C) at the time of contacting the mixture of a water-miscible organic solvent (B) and water (C) with the coffee bean extract (A), is preferably 1.2% or greater, and it is more preferable to adjust the content in the range of 2% or greater, and even more preferably in the range of 4% or greater. As for the method of adjusting the content in the above range, it is preferable to measure the contents of the chlorogenic acids, and the components (B) and (C) in the coffee bean extract (A) in advance, and to adjust the amount of use of the mixture of components (B) and (C) such that the amount of chlorogenic acids falls in a predetermined range during the treatment.

Here, the disagreeable taste and unpleasant odor may be exemplified by a burnt odor or bitter taste originating from roasted coffee beans, an acrid taste and grassy flavor originating from green coffee beans, or the like. Roasted coffee has a characteristic burnt odor or bitter taste, and thus is highly favored. However, when roasted coffee beans themselves or an extract thereof is to be incorporated into other food products, the taste and flavor give an unpleasant sensation, and thus incorporation thereof may not be preferable depending on the type of food products. Furthermore, since green coffee beans have a characteristic acrid taste or grassy odor, when green coffee beans themselves or an extract thereof is incorporated directly into food products and the like, the characteristic taste and odor become obstacles.

In the case of contacting the coffee bean extract (A) with a mixture of the water-miscible organic solvent (B) and water (C), the temperature is preferably set at 25 to 90°C from the viewpoints of the duration of treatment or treatment efficiency and the recovery rate of chlorogenic acids, and it is more preferable to set the temperature at 45 to 70°C from the viewpoints of improving the recovery rate of chlorogenic acids and the efficiency of removing disagreeable taste and unpleasant odor. In addition, it is preferable to set the duration of treatment appropriately in balance with the treatment temperature.

According to the present invention, from the viewpoints of improving the efficiency of removing the disagreeable taste and unpleasant odor components, it is preferable to use a solid powder as a dispersant at the time of contacting the coffee bean extract (A) with a mixture of the water-miscible organic solvent (B) and water (C), because the solid portion in a coffee bean extract is prevented from aggregating (becoming taffy), and the solid-liquid interface increases, thus making the dispersibility good. It is preferable to add the dispersant at the beginning of contacting a mixture of the water-miscible organic solvent (B) and water (C) with the component (A), and it is more preferable to mix the dispersant with a mixture of the components (B) and (C) in advance, and then to add the coffee bean extract (A) to cause the components to contact, from the viewpoints of obtaining high dispersing effects and effectively removing disagreeable taste and unpleasant odor. At the time of adding to the mixture of the components (B) and (C), the component (A) may be a paste-like concentrate or may be powdered. Furthermore, if the component (A) is a powdered product, the component may be mixed with a dispersant in advance, and then may be dispersed in a mixture of the components (B) and (C). The dispersant which can be used in the .present invention may be a powdered material, and diatomaceous earth, white clay and the like can be used. Specifically, Silika 100F-A (Chuo Silika Co., Ltd.), which is a type of diatomaceous earth, and the like may be mentioned. The average particle size of the dispersant is preferably in the range of 0.1 to 100 µm, and more preferably in the range of 5 to 20 µm. The amount of addition of the dispersant is preferably adjusted to 5 to 200 parts by mass (hereinafter, simply referred to as "parts"), more preferably 10 to 100 parts, and even more preferably 30 to 70 parts, relative to 100 parts of the coffee bean extract (A), from the viewpoint of the efficiency of removing disagreeable taste and unpleasant odor.

It is preferable for the coffee bean extract which has been subjected to a contact with a mixture of the water-miscible organic solvent (B) and water (C), to have the liquid phase removed by solid-liquid separation such as filtration, and to have the recovered solid portion washed with a liquid, if necessary. The liquid used in the washing is preferably the water-miscible organic solvent (B), or a mixture of the water-miscible organic solvent (B) and water (C) as used in the contact treatment, from the viewpoint of improving the recovery rate of chlorogenic acids.

The recovered solid portion is made into a chlorogenic acid-containing composition which has less disagreeable taste and less unpleasant odor and contains chlorogenic acids at a high concentration, by treating the solid portion with a solubilizing liquid as defined below; extracting or dissolving a soluble fraction of the solid portion in the solubilizing liquid; and performing filtration, concentration, drying or the like, as necessary. Particularly, in the case of having used a dispersant which is insoluble in the solubilizing liquid, it is preferable to perform filtration from the viewpoint of separating the dispersant.

As for the solubilizing liquid used in this case, water may be used singly; however, since darkening of the color phase occurs in accordance with the type of the dispersant used, in the case of using a dispersant, it is preferable to use a mixture of a water-miscible organic solvent (B) and water (C). In this case, the mass ratio of the mixture of a water-miscible solvent (B) and water (C) is set to 5/95 or greater and less than 70/30, more preferably 5/95 to 60/40, even more preferably 10/90 to 50/50, and even more preferably 20/80 to 40/60, from the viewpoints of improving the recovery rate of chlorogenic acids and of the color phase.

The method of the present invention can remove disagreeable taste and unpleasant odor from a coffee bean extract, and at the same time, can also remove caffeine therefrom to some extent. As for the extent, the mass ratio of caffeine/chlorogenic acids can be 10 to 60% of the mass ratio before treating by the method of the present invention. Furthermore, in the case of decaffeinating to a high extent, it is preferable that the separate, above-mentioned recovered solid portion is contacted with activated carbon when the solid portion is dissolved in a mixture of the water-miscible solvent (B)/water (C) having a mass ratio of the components of 5/95 or greater and less than 70/30, or that the chlorogenic acid-containing material is dissolved in the same mixture and then contacted with activated carbon. Here, it is preferable to add the mixture of the components (B) and (C) in an amount of 250 to 1000 parts, and more preferably 300 to 700 parts, relative to 100 parts of the chlorogenic acid-containing material, from the viewpoint of the efficiency of removing caffeine.

In the case of performing a separate decaffeinating operation, it is preferable to add a mixture of the components (B) and (C) dropwise to the chlorogenic acid-containing material in a stirred state so as to increase the efficiency of extracting chlorogenic acids. After completion of the dropwise addition of the mixture of components (B) and (C), it is further preferable to provide an aging time of about 10 to 120 minutes. These treatments can be conducted at 10 to 60°C, and it is particularly preferable to conduct the treatments at 10 to 50°C, and further preferably at 10 to 40°C.

In the case of performing a separate decaffeinating operation, the activated carbon used is not particularly limited as long as it is generally used at an industrial level, and for example, commercially available products such as ZN-50 (manufactured by Hokuetsu Carbon Industry Co., Ltd.), Kuraraycoal GLC, Kuraraycoal PK-D, Kuraraycoal PW-D (manufactured by Kuraray Chemical Co., Ltd.), Shirasagi AW50, Shirasagi A, Shirasagi M, Shirasagi C, and Shirasagi WH2C (manufactured by Japan EnviroChemicals, Ltd.) can be used. The pore volume of the activated carbon is preferably 0.01 to 0.8 mL/g, and more preferably 0.1 to 0.7 mL/g. The specific surface area is preferably in the range of 800 to 1300 m²/g, and more preferably 900 to 1200 m²/g. In addition, these property values are values obtained on the basis of the nitrogen adsorption method. The amount of use of the activated carbon is preferably 5 to 50 parts, and more preferably 10 to 30 parts relative to 100 parts of the component (A), from the viewpoint of the efficiency of removing caffeine and the efficiency of filtration.

In the case of performing a separate decaffeinating operation, it is preferable to conduct a treatment with activated carbon, and at the same time or in place thereof, to conduct a treatment with acidic white clay or activated white clay, from the viewpoint of removing disagreeable taste and unpleasant odor. Both of acidic white clay and activated white clay may contain SiO₂, Al₂O₃, Fe₂O₃, CaO, MgO and the like as general chemical components, but one such clay having a mass ratio of SiO₂/Al₂O₃ of 3 to 12, and particularly 4 to 9, is preferred. Furthermore, one such clay having a composition containing 2 to 5% of Fe₂O₃, 0 to 1.5% of CaO and 1 to 7% of MgO is preferred. The activated white clay is a product obtained by treating naturally occurring acidic white clay (montmorillonite clay) with a mineral acid such as sulfuric acid, and is a compound having a porous structure with a large specific surface area and large adsorption capacity. It is known that when the acidic white clay is further treated with an acid, the specific surface area changes, thus resulting in an improvement of the decolorization capacity and changes in properties.

The specific surface area of the acidic white clay or activated white clay may vary depending on the extent of the acid treatment, or the like, but is preferably 50 to 350 m²/g. The pH value (5% suspension) of the clay is preferably 2.5 to 8, and particularly preferably 3.6 to 7. For example, as the acidic white clay, commercially available products such as Mizuka-Ace #600 (manufactured by Mizusawa Industrial Chemicals, Ltd.) can be used.

The acidic white clay or activated white clay is preferably added in an amount of 5 to 50 parts, and more preferably 10 to 30 parts, relative to 100 parts of the component (A), from the viewpoints of the efficiency of removing caffeine and the cake resistance in the filtration process.

The treatment of contacting a solution prepared by dissolving the chlorogenic acid-containing material in the mixture of components (B) and (C), with activated carbon and/or acidic white clay or activated white clay, may be performed by any of batch type methods, continuous processing using columns and the like. In general, a method of adding powdered activated carbon while stirring for selectively adsorption of caffeine, and then obtaining a filtrate having caffeine removed by a filtering operation; or a method of selectively adsorbing caffeine by a continuous treatment using a column packed with granular activated carbon or the like, is employed.

Subsequently, from the solution of chlorogenic acid-containing material after being contacted with activated carbon and/or acidic white clay or activated white clay, the water-miscible organic solvent (B) and water (C) are distilled off using a method such as distillation under reduced pressure. The chlorogenic acid-containing material after the treatment may be in the liquid state or in the solid state, and may also be powdered according to a method such as freeze-drying or spray drying.

The chlorogenic acid-containing material obtained by the methodof the present invention richly contains chlorogenic acids in an amount of 10 to 70%, preferably 20 to 60%, and more preferably 30 to 50%, in the dry solid fraction of the chlorogenic acid-containing material, and thus the chlorogenic acid-containing material is optimally processed into a food product having an antihypertensive action, a food product indicated as intended to have an antihypertensive action, a food product indicated to be adequate for high blood pressure, or the like. Examples of the food product include confectionaries such as jelly and cake; beverages such as juice, coffee, tea, sports drinks and soup; seasonings such as miso paste, soy sauce, thin soup and marinade; and the like; however, from the viewpoints of having a high absorption efficiency and effectively manifesting an antihypertensive action, liquid food products are preferred, and from the same viewpoints, it is preferable to incorporate the chlorogenic acid-containing material into food products which can be taken every day, such as beverages and seasonings. It is preferable that the amount of incorporation of the chlorogenic acid-containing material in the food products per meal is 10 to 5000 mg, and more preferably 50 to 2000 mg, from the viewpoint of effectively manifesting the antihypertensive action.

Furthermore, since the chlorogenic acid-containing material obtained by the method of the present invention has a less disagreeable taste and less unpleasant odor and has a pleasant flavor, the material does not exert any influence on the quality of food products, even if incorporated into various foods. For that reason, the chlorogenic acid-containing material can also be preferably incorporated into, among the above-mentioned food products, beverages having a bland taste or seasonings having subtle influence on the daintiness of foods .

### [EXAMPLES]

### [Method for measuring chlorogenic acid content and caffeine content]

The method for measuring the chlorogenic acid content and the caffeine content is as follows.

### (Analytical instrument)

An HPLC (manufactured by Shimadzu Corporation.) was used. The model numbers of the constituent units of the apparatus are as follows. Detector: SPD-M10A, Oven: CTO-10AC, Pump: LC-10AD, Autosampler: SIL-10AD, Column: Inertsil ODS-2 (internal diameter 4.6 mm × length 250 mm)

### (Analysis conditions)

Amount of sample injection: 10 µL, Flow rate: 1.0 mL/min, Detection wavelength for ultraviolet absorptiometer: 325 nm (chlorogenic acids), 270 nm (caffeine), Eluent A: 0.05 M acetic acid-3% acetonitrile solution, Eluent B: 0.05 M acetic acid-100% acetonitrile solution

### (Concentration gradient conditions)

| Time | Eluent A | Eluent B |
|---|---|---|
| 0 min | 100% | 0% |
| 20 min | 80% | 20% |
| 35 min | 80% | 20% |
| 45 min | 0% | 100% |
| 60 min | 0% | 100% |
| 70 min | 100% | 0% |
| 120 min | 100% | 0% |

### (Retention times of chlorogenic acids)

3-Caffeoylquinic acid (3-CQA): 16.8 min
5-Caffeoylquinic acid (5-CQA): 19.8 min
4-Caffeoylquinic acid (4-CQA): 21.5 min
3-Ferulylquinic acid (3-FQA): 22.2 min
5-Ferulylquinic acid (5-FQA): 26.1 min
4-Ferulylquinic acid (4-FQA): 27.2 min
3,5-Dicaffeylquinic acid (3,5-diCQA): 33.5 min
3,4-Dicaffeylquinic acid (3,4-diCQA): 33.8 min
4,5-Dicaffeylquinic acid (4,5-diCQA): 36.0 min

The percent by mass values were determined from the percent by area values determined herein, using 5-CQA as a standard material.

### (Retention time of caffeine)

### 19.4 min

The percent by mass value was determined from the percent by area value determined herein, using reagent-grade caffeine as a standard material. Additionally, an evaluation of the effects of removing caffeine was performed on the basis of the mass ratio of caffeine/chlorogenic acids.

### [Methods for measurements of recovery rate for chlorogenic acids]

Taking the content of chlorogenic acids contained in green beans as 100%, the recovery rate of chlorogenic acids which could be recovered from the coffee bean extract, and the recovery rate for chlorogenic acids which could be finally recovered as the chlorogenic acid-containing material produced by the method of the present invention were evaluated. That is, the recovery rate of chlorogenic acids was calculated by subtracting the total amount of chlorogenic acids finally recovered from the content of chlorogenic acids contained in green beans, and the recovery rate was evaluated on the basis of the following criteria.
A: The recovery rate of chlorogenic acids is 60% or greater and less than 100%.
B: The recovery rate of chlorogenic acids is 45% or greater and less than 60%.
C: The recovery rate of chlorogenic acids is 30% or greater and less than 45%.
D: The recovery rate of chlorogenic acids is 15% or greater and less than 30%.
E: The recovery rate of chlorogenic acids is 0% or greater and less than 15%.

### [Method for producing coffee bean extract]

### (1) Green coffee bean extract S

500 g of Vietnamese Robusta G-1 green beans was subjected to extraction while stirring in 5 L of hot water at 98°C for 4 hours. After cooling, solid-liquid separation was performed, and the extract was concentrated under reduced pressure at 40°C until the solids concentration reached 20 w/v%. Then, the concentrated extract was spray dried to prepare a green coffee bean extract S. The content of chlorogenic acids was 38.1% by mass. The recovery rate for chlorogenic acids in the course of extraction was 80%.

### (2) Coffee bean extract T

500 g of Vietnamese Robusta G-1 roasted beans which had been roasted until the L value reached 35, was subjected to extraction while stirring in 5 L of hot water at 98°C for 4 hours. After cooling, solid-liquid separation was performed, and the extract was concentrated under reduced pressure at 40°C until the solids concentration reached 20 w/v%. Then, the concentrated extract was spray dried to prepare a 35-L value coffee bean extract T. The content of chlorogenic acids was 24.1% by mass. The recovery rate for chlorogenic acids in the course of roasting and extraction, as compared with the chlorogenic acid content in green beans, was 53%.

### (3) Coffee bean extract U

500 g of Colombian Arabica roasted beans which had been roasted until the L value reached 18, was subjected to extraction while stirring in 5 L of hot water at 98°C for 4 hours. After cooling, solid-liquid separation was performed, and the extract was concentrated under reduced pressure at 40°C until the solids concentration reached 20 w/v%. Then, the concentrated extract was spray dried to prepare an 18-L value coffee bean extract U. The content of chlorogenic acids was 4.5% by mass. The recovery rate for chlorogenic acids in the course of roasting and extraction, as compared with the chlorogenic acid content in green beans, was 24%.

In addition, the content of chlorogenic acids in commercially available instant coffee X (Nescafe Gold Blend) was measured, and was found to be 4.0% by mass.

### [Method for measuring L value of roasted coffee beans]

10 g of roasted coffee beans was ground using a High Cut Coffee Mill (Kalita Co., Ltd.) with gauge 1, and the measurement was performed with a colorimeter, such as Spectro Color Meter SE2000 (Nippon Denshoku Industries Co., Ltd.). Additionally, three measurements were made for one sample, and the average value was taken as the L value.

### Example 1-(1) and (2)

70 g of the green coffee bean extract S, and 280 g of a solvent mixture of ethanol and water (ethanol/water mass ratio as indicated in Table 1) were mixed and brought into contact by shaking at 60°C (or 50°C) for 3 hours. The liquid portion was removed by means of Nutsche filtration (No. 2 filter paper used), and then while still maintaining the Nutsche suction, cake washing of the solid portion was performed using 280 g of 99.5% ethanol. The solid portion was dissolved in 300 g of a solvent mixture of ethanol and water (ethanol/water mass ratio = 30/70), and was subjected again to Nutsche filtration (No. 2 filter paper used) . Then, cake washing of the solid portion was performed using 300 g of the same solvent, to recover a soluble fraction. The recovered fraction was concentrated to about 55% of the dry solid portion in an evaporator. Thus, chlorogenic acid-containing materials I- (1) and (2) according to the method of the present invention were obtained.

### Example 2-(1) to (8)

70 g of the green coffee bean extract S, 280 g of a solvent mixture of ethanol and water (ethanol/water mass ratio as indicated in Table 1), and 35 g of a dispersant (Silika 100F-A) were mixed and brought into contact by shaking at 60°C for 3 hours. The liquid portion was removed by means of Nutsche filtration (No. 2 filter paper used), and then while still maintaining the Nutsche suction, cake washing of the solid portion was performed using 280 g of 99.5% ethanol. The solid portion was dissolved in 300 g of a solvent mixture of ethanol and water (ethanol/water mass ratio = 30/70), and was subjected again to Nutsche filtration (No. 2 filter paper used). Then, cake washing of the solid portion was performed using 300 g of the same solvent, to recover a soluble fraction. The recovered fraction was concentrated to about 55% of the dry solid portion in an evaporator. Thus, chlorogenic acid-containing materials II- (1) to (8) according to the method of the present invention were obtained.

### Example 3-(1) to (4)

The green coffee bean extract S in an amount as indicated in Table 1, a solvent mixture of ethanol and water in an amount as indicated in Table 1 (ethanol/water mass ratio as indicated in Table 1), and a dispersant (Silika 100F-A) in an amount as indicated in Table 1 were mixed and brought into contact by shaking at 60°C for 3 hours. The liquid portion was removed by means of Nutsche filtration (No. 2 filter paper used), and then while still maintaining the Nutsche suction, cake washing of the solid portion was performed using 99.5% ethanol in an amount 4-fold larger than the mass of the coffee bean extract S. The solid portion was dissolved in 300 g of a solvent mixture of ethanol and water (ethanol/water mass ratio = 30/70), and was subjected again to Nutsche filtration (No. 2 filter paper used). Then, cake washing of the solid portion was performed using 300 g of the same solvent, to recover a soluble fraction. The recovered fraction was concentrated to about 55% of the dry solid portion in an evaporator. Thus, chlorogenic acid-containing materials III- (1) to (4) according to the method of the present invention were obtained.

### Example 4-(1) and (2)

70 g of the coffee bean extract T, 280 g of a solvent mixture of ethanol and water (ethanol/water mass ratio as indicated in Table 1), and 35 g of a dispersant (Silika 100F-A) were mixed and brought into contact by shaking at 60°C for 3 hours. The liquid portion was removed by means of Nutsche filtration (No. 2 filterpaper used), and then while still maintaining theNutsche suction, cake washing of the solid portion was performed using 280 g of 99.5% ethanol. The solid portion was dissolved in 300 g of a solvent mixture of ethanol and water (ethanol/water mass ratio = 30/70), and was subjected again to Nutsche filtration (No. 2 filter paper used). Then, cake washing of the solid portion was performed using 300 g of the same solvent, to recover a soluble fraction. The recovered fraction was concentrated to about 55% of the dry solid portion in an evaporator. Thus, chlorogenic acid-containing materials IV-(1) and (2) according to the method of the present invention were obtained.

### Example 5-(1) to (4)

The coffee bean extract U in an amount as indicated in Table 1, a solvent mixture of ethanol and water in an amount as indicated in Table 1 (ethanol/water mass ratio as indicated in Table 1), and a dispersant (Silika 100F-A) in an amount as indicated in Table 1 were mixed and brought into contact by shaking at 60°C for 3 hours. The liquid portion was removed by means of Nutsche filtration (No. 2 filter paper used), and then while still maintaining the Nutsche suction, cake washing of the solid portion was performed using 99.5% ethanol in an amount 4-fold larger than the mass of the coffee bean extract U. The solid portion was dissolved in 300 g of a solvent mixture of ethanol and water (ethanol/water mass ratio = 30/70), and was subjected again to Nutsche filtration (No. 2 filter paper used) . Then, cake washing of the solid portion was performed using 300 g of the same solvent, to recover a soluble fraction. The recovered fraction was concentrated to about 55% of the dry solid portion in an evaporator. Thus, chlorogenic acid-containing materials V- (1) to (4) according to the method of the present invention were obtained.

### Example 6

29.5 g of instant coffee powder X, 125 g of a solvent mixture of ethanol and water (ethanol/water mass ratio as indicated in Table 1), and 14.8 g of a dispersant (Silika 100F-A) were mixed and brought into contact by shaking at 60°C for 3 hours. The liquid portion was removed by means of Nutsche filtration (No. 2 filter paper used), and then while still maintaining the Nutsche suction, cake washing of the solid portion was performed using 120 g of 99.5% ethanol. The solid portion was dissolved in 100 g of a solvent mixture of ethanol and water (ethanol/water mass ratio = 30/70), and was subjected again to Nutsche filtration (No. 2 filter paper used). Then, cake washing of the solid portion was performed using 100 g of the same solvent, to recover a soluble fraction. The recovered fraction was concentrated to about 55% of the dry solid portion in an evaporator. Thus, a chlorogenic acid-containing material VII according to the method of the present invention was obtained. Additionally, in the present Example, although the recovery rate of chlorogenic acids from green beans could not be quantified, since the degree of roasting of conventional coffee for drinking, that is, the L value, is expected to be about 15 to 20, the recovery rate of chlorogenic acids from green beans was estimated to be of approximately the same extent as those of Example 5- (1), (2) and Comparative Example 3.

### Example 7

400 g of the green coffee bean extract S, 1600 g of a solvent mixture of ethanol and water (ethanol/water mass ratio = 92.4/7.6), and 200 g of a dispersant (Silika 100F-A) were mixed and brought into contact by shaking at 60°C for 3 hours. The liquid portion was removed by means of Nutsche filtration (No. 2 filter paper used), and then while still maintaining the Nutsche suction, cake washing of the solid portion was performed using 1600 g of 99.5% ethanol. The amount of recovered solid portion was 1141 g. 600 g of the solid portion was dissolved in 500 g of a solvent mixture of ethanol and water (ethanol/water mass ratio = 20/80), and was subjected again to Nutsche filtration (No. 2 filter paper used). Then, cake washing of the solid portion was performed using 200 g of a solvent mixture of ethanol and water (ethanol/water mass ratio = 50/50), to recover 973 g of a soluble fraction. 350 g of the recovered soluble fraction was collected by preparative isolation, and was passed through a column packed with 12 g of activated carbon Shirasagi WH2C (manufactured by Japan EnviroChemicals, Ltd.) at a flow rate of 1 g/min at room temperature, to perform a caffeine removal treatment. As a result, the mass ratio of caffeine/chlorogenic acids in the soluble fraction was 0.103 before the treatment with activated carbon, while the ratio was 0.026 after the treatment with activated carbon. Furthermore, the yield of chlorogenic acids in the activated carbon treatment process was 91.1%. From the above results, it was confirmed that when the chlorogenic acid-containing material to which the method of the present invention has been applied, is subjected to a decaffeination treatment, caffeine can be efficiently removed without reducing the content of chlorogenic acids.

### Comparative Examples 1 to 4

Chlorogenic acid-containing materials which were not based on the method of the present invention were defined as follows.
Comparative Example 1: Green coffee bean extract S
Comparative Example 2: Coffee bean extract T
Comparative Example 3: Coffee bean extract U
Comparative Example 4: Commercially available instant coffee X

### [Sensory evaluation]

With respect to each of the chlorogenic acid-containing materials obtained in Examples and Comparative Examples, an aqueous solution at a chlorogenic acid concentration of 1600 mg/L was prepared, and the intensity of the flavor at the moment when the aqueous solution was held in the mouth was subjected to a sensory evaluation. In addition, the evaluation of the chlorogenic acid-containing material produced by the method of the present invention was performed on the basis of the following evaluation criteria, while taking those chlorogenic acid-containing materials which were prepared for the respective samples, not by employing the method of the present invention, as control samples. That is, the green coffee bean extract S was taken as the control for the chlorogenic acid-containing materials I to IV; the coffee bean extract T was taken as the control for the chlorogenic acid-containing material V; the coffee bean extract U was taken as the control for the chlorogenic acid-containing material VI; and the commercially available instant coffee X was taken as the control for the chlorogenic acid-containing material VII respectively.

### [Evaluation criteria on acrid taste/grassy odor originating from green coffee beans or green coffee bean extract]

A: Virtually no acrid taste/grassy odor is felt.
B: Acrid taste/grassy odor has been reduced by a large extent.
C: Acrid taste/grassy odor has been reduced.
D: Acrid taste/grassy odor has been slightly reduced.
E: Samples have acrid taste/grassy odor which is equivalent to the taste/odor of the controls.

### [Evaluation criteria on burnt odor/bitter taste originating from roasted coffee beans or roasted coffee bean extract]

A: Virtually no burnt odor/bitter taste is felt.
B: Burnt odor/bitter taste has been reduced by a large extent.
C: Burnt odor/bitter taste has been reduced.
D: Burnt odor/bitter taste has been slightly reduced.
E: Samples have burnt odor/bitter taste which is equivalent to the odor/taste of the controls.

**[Table 1]**

| | | | Raw material (A) of method of the invention | | | Water-miscible solvent (B)/Water (C) | | | Content of chlorogenic acids in total of components (A), (B) and (C) (% by mass) ³⁾ | Dispersant (g) |
|---|---|---|---|---|---|---|---|---|---|---|
| | | | Type | Amount of use (g) | Water content (% by mass) | Ratio of incorporated masses ¹⁾ | Amount of use (g) | mass ratio after mixing ²⁾ | | |
| Example | 1 | (1) | Green coffee bean extract S | 70 | 4.6 | 95.6 / 4.4 | 280 | 94.5 / 5.5 | 7.61 | - |
| | | (2) | | 70 | 4.6 | 97.0/ 3.0 | 280 | 95.9 / 4.1 | 7.61 | - |
| | 2 | (1) | Green coffee bean extract S | 70 | 4.6 | 75.0 / 25.0 | 280 | 74.1 / 25.9 | 7.61 | 35 |
| | | (2) | | 70 | 4.6 | 85.0/ 15.0 | 280 | 84.0 / 16.0 | 7.61 | 35 |
| | | (3) | | 70 | 4.6 | 90.0 / 10.0 | 280 | 89.0 / 11.0 | 7.61 | 35 |
| | | (4) | | 70 | 4.6 | 92.4 / 7.6 | 280 | 91.3 / 8.7 | 7.61 | 35 |
| | | (5) | | 70 | 4.6 | 95.4 / 4.6 | 280 | 94.5 / 5.5 | 7.61 | 35 |
| | | (6) | | 70 | 4.6 | 97.0 / 3.0 | 280 | 95.9 / 4.1 | 7.61 | 35 |
| | | (7) | | 70 | 4.6 | 98.0 / 2.0 | 280 | 96.9 / 3.1 | 7.61 | 35 |
| | | (8) | | 70 | 4.6 | 99.5 / 0.5 | 280 | 98.4 / 1.6 | 7.61 | 35 |
| | 3 | (1) | Green coffee bean extract S | 52.5 | 4.6 | 92.4/ 7.6 | 298 | 91.7 / 8.3 | 5.7 | 26.3 |
| | | (2) | | 35 | 4.6 | 92.4/ 7.6 | 315 | 91.9 / 8.1 | 3.8 | 17.5 |
| | | (3) | | 17.5 | 4.6 | 92.4/ 7.6 | 333 | 92.2 / 7.8 | 1.9 | 8.8 |
| | | (4) | | 10.5 | 4.6 | 92.4/ 7.6 | 340 | 92.3 / 7.7 | 1.1 | 5.3 |
| | 4 | (1) | Coffee bean extract T | 70 | 5.0 | 92.4/ 7.6 | 280 | 91.3 / 8.7 | 4.82 | 35 |
| | | (2) | | 70 | 5.0 | 95.6 / 4.4 | 280 | 94.4 / 5.6 | 4.82 | 35 |
| | 5 | (1) | Coffee bean extract U | 105 | 5.3 | 92.4/ 7.6 | 245 | 90.3 / 9.7 | 1.34 | 53 |
| | | (2) | | 70 | 5.3 | 95.6 / 4.4 | 280 | 94.3 / 5.7 | 0.89 | 35 |
| | | (3) | | 105 | 5.3 | 95.6 / 4.4 | 245 | 93.5 / 6.5 | 1.34 | 53 |
| | | (4) | | 122.5 | 5.3 | 95.6 / 4.4 | 228 | 93.0 / 7.0 | 1.56 | 61.3 |
| | 6 | | Commercially available instant coffee X | 29.5 | 6.0 | 92.4/ 7.6 | 125 | 91.1 / 8.9 | 0.76 | 14.8 |

**[Table 2]**

| | | | Chlorogenic acid-containing material | | Flavor evaluation | | Recovery rate of chlorogenic acids | | Caffeine/chloro genie acids (mass ratio) |
|---|---|---|---|---|---|---|---|---|---|
| | | | | | Acrid taste/grassy odor | Burnt odor/bitter taste | Evaluation | (%) | |
| | 1 | (1) | I | (1) | B | A | A | 70.4 | 0.095 |
| | | (2) | | (2) | B | A | A | 70.4 | 0.098 |
| | 2 | (1) | II | (1) | C | A | E | 17.6 | 0.130 |
| | | (2) | | (2) | B | A | B | 49.6 | 0.130 |
| | | (3) | | (3) | B | A | A | 63.2 | 0.149 |
| | | (4) | | (4) | A | A | A | 65.6 | 0.116 |
| | | (5) | | (5) | A | A | A | 68.8 | 0.113 |
| | | (6) | | (6) | B | A | A | 71.2 | 0.106 |
| | | (7) | | (7) | C | A | A | 73.6 | 0.130 |
| | | (8) | | (8) | D | A | A | 69.6 | 0.132 |
| Example | 3 | (1) | III | (1) | A | A | A | 63.2 | 0.097 |
| | | (2) | | (2) | B | A | B | 53.6 | 0.078 |
| | | (3) | | (3) | C | A | C | 32.0 | 0.050 |
| | | (4) | | (4) | C | A | D | 16.8 | 0.033 |
| | 4 | (1) | IV | (1) | A | B | D | 42.7 | 0.147 |
| | | (2 | | (2) | A | B | D | 44.3 | 0.144 |
| | 5 | (1) | V | (1) | A | D | E | 20.1 | 0.295 |
| | | (2) | | (2) | A | D | E | 18.9 | 0.158 |
| | | (3) | | (3) | A | D | E | 20.1 | 0.270 |
| | | (4) | | (4) | A | D | E | 21.1 | 0.327 |
| | 6 | | VI | | A | D | (E) | - | 0.082 |
| Comparative Example | 1 | | Green coffee beans | | E | A | - | 80.0 | 0.262 |
| | 2 | | Coffee bean extract T | | A | E | - | 52.7 | 0.385 |
| | 3 | | Coffee bean extract U | | A | E | - | 24.0 | 0.981 |
| | 4 | | Commercially available instant coffee | | A | E | (E) | - | 0.669 |

As is obvious from the results of Table 1 and Table 2, a chlorogenic acid-containing material having less disagreeable taste and less unpleasant odor can be obtained using the method of the present invention.

## Claims

1. A method for production of a chlorogenic acid-containing material, comprising contacting a coffee bean extract (A), which has been extracted from coffee beans using water or a mixture of a water miscible organic solvent and water at a mass ratio of water-miscible organic solvent/water of less than 70/30,
with a mixture of a water-miscible organic solvent (B) and water (C), under such conditions that the mass ratio of the components (B) and (C) obtained after the mixture is mixed with the component (A) becomes 70/30 to 99.5/0.5;
recovering a solid portion; and
adding water (C) or a mixture having a mass ratio of water-miscible solvent (B)/ water (C) of 5/95 or greater and less than 70/30 to the recovered solid portion, to dissolve the solid portion or to extract chlorogenic acids.

2. The method for production of a chlorogenic acid-containing material according to claim 1, wherein the coffee bean extract (A) is originating from green coffee beans or lightly roasted coffee beans having an L value of 30 or greater.

3. The method for production of a chlorogenic acid-containing material according to claim 1 or 2, wherein the content of chlorogenic acids contained in the total of the components (A), (B) and (C), upon contacting the coffee bean extract (A) with a the mixture of the water-miscible organic solvent (B) and water (C), is 1.2% by mass or greater.

4. The method for production of a chlorogenic acid-containing material according to any one of claims 1 to 3, wherein the water-miscible solvent (B) is an alcohol.

5. The method for production of a chlorogenic acid-containing material according to any one of claims 1 to 4, wherein a powdered solid is added as a dispersant upon performing the contact with the mixture of the water-miscible solvent (B) and water (C); and the solid portion is recovered later.

6. Method for preparing a food product containing 0.005 to 10% by mass of chlorogenic acids, the method comprising the steps of:
producing the chlorogenic acid-containing material by the method according to any one of claims 1 to 5; and
incorporating the chlorogenic acid-containing material into a food product.

## Patentansprüche

1. Verfahren zur Herstellung eines Chlorogensäure-haltigen Materials, umfassend das Inkontaktbringen eines Kaffeebohnenextrakts (A), der aus Kaffeebohnen unter Verwendung von Wasser oder einer Mischung von einem wassermischbaren organischen Lösungsmittel und Wasser in einem Massenverhältnis von dem wassermischbaren organischen Lösungsmittel/Wasser von weniger als 70/30 extrahiert wurde,
mit einer Mischung von einem wassermischbaren organischen Lösungsmittel (B) und Wasser (C) unter solchen Bedingungen, dass das Massenverhältnis der Komponenten (B) und (C), erhalten nach dem Mischen der Mischung mit der Komponente (A), 70/30 bis 99,5/0,5 wird;
das Wiedergewinnen eines Feststoffanteils; und
das Hinzufügen von Wasser (C) oder einer Mischung mit einem Massenverhältnis von wassermischbarem Lösungsmittel (B)/Wasser (C) von 5/95 oder mehr und weniger als 70/30 zu dem wiedergewonnenen Feststoffanteil, um den Feststoffanteil aufzulösen oder Chlorogensäuren zu extrahieren.

2. Verfahren zur Herstellung eines Chlorogensäure-haltigen Materials gemäß Anspruch 1, bei dem der Kaffeebohnenextrakt (A) aus grünen Kaffeebohnen oder leicht gerösteten Kaffeebohnen mit einem L-Wert von 30 oder mehr stammt.

3. Verfahren zur Herstellung eines Chlorogensäure-haltigen Materials gemäß Anspruch 1 oder 2, bei dem der Gehalt an Chlorogensäuren, der in der Gesamtmenge der Komponenten (A), (B) und (C) nach Inkontaktbringen des Kaffeebohnenextrakts (A) mit der Mischung von dem wassermischbaren organischen Lösungsmittel (B) und Wasser (C) enthalten ist, 1,2 Massen-% oder mehr ist.

4. Verfahren zur Herstellung eines Chlorogensäure-haltigen Materials gemäß einem der Ansprüche 1 bis 3, bei dem das wassermischbare Lösungsmittel (B) ein Alkohol ist.

5. Verfahren zur Herstellung eines Chlorogensäure-haltigen Materials gemäß einem der Ansprüche 1 bis 4, bei dem ein pulverförmiger Feststoff als Dispergiermittel bei der Durchführung des Inkontaktbringens mit der Mischung von dem wassermischbaren Lösungsmittel (B) und Wasser (C) zugegeben wird; und der Feststoffanteil später wiedergewonnen wird.

6. Verfahren zur Herstellung eines Lebensmittelprodukts, enthaltend 0,005 bis 10 Massen-% Chlorogensäuren, wobei das Verfahren die folgenden Schritte umfasst:
das Herstellen des Chlorogensäure-haltigen Materials gemäß dem Verfahren gemäß einem der Ansprüche 1 bis 5; und
das Einbringen des Chlorogensäure-haltigen Materials in ein Nahrungsmittelprodukt.

## Revendications

1. Procédé de production d'un matériau contenant de l'acide chlorogénique, comprenant la mise en contact d'un extrait de grain de café (A), qui a été extrait de grains de café en utilisant de l'eau ou un mélange d'un solvant organique miscible dans l'eau et d'eau à un rapport en masse de solvant organique miscible dans l'eau/eau inférieur à 70/30,
avec un mélange d'un solvant organique miscible dans l'eau (B) et d'eau (C), dans de telles conditions que le rapport en masse des composants (B) et (C) obtenu après que le mélange est mélangé avec le composant (A) devienne 70/30 à 99,5/0,5 ;
la récupération d'une portion solide ; et
l'addition d'eau (c) ou d'un mélange ayant un rapport en masse de solvant miscible dans l'eau (B)/eau (C) de 5/95 ou supérieur et inférieur à 70/30 à la portion solide récupérée, pour dissoudre la portion solide ou pour extraire des acides chlorogéniques.

2. Le procédé de production d'un matériau contenant de l'acide chlorogénique selon la revendication 1, dans lequel l'extrait de grain de café (A) provient de graines de café vertes ou des graines de cafés légèrement torréfiées ayant une value L de 30 ou supérieure.

3. Le procédé de production d'un matériau contenant de l'acide chlorogénique selon la revendication 1 ou 2, dans lequel la teneur d'acides chlorogéniques contenus dans le total des composants (A), (B) et (C), lors de la mise en contact de l'extrait de graine de café (A) avec le mélange du solvant organique miscible dans l'eau (B) et d'eau (C), est 1,2% en masse ou supérieure.

4. Le procédé de production d'un matériau contenant de l'acide chlorogénique selon l'une quelconque des revendications 1 à 3, dans lequel le solvant miscible dans l'eau (B) est un alcool.

5. Le procédé de production d'un matériau contenant de l'acide chlorogénique selon l'une quelconque des revendications 1 à 4, dans lequel u solide en poudre est ajouté en tant que dispersant lors de la réalisation de la mise en contact avec le mélange du solvant miscible dans l'eau (B) et d'eau (C) ; et la portion solide est récupérée plus tard.

6. Procédé de préparation d'un produit alimentaire contenant de 0,005 à 10% en masse d'acides chlorogéniques, le procédé comprenant les étapes de :
production du matériau contenant de l'acide chlorogénique par le procédé selon l'une quelconque des revendications 1 à 5 ; et
incorporation du matériau contenant de l'acide chlorogénique dans un produit alimentaire.
